# FASCICULE DE BREVET EUROPEEN

(11) **EP 0 444 102 B1**
(45) Date de publication et mention de la délivrance du brevet: **28.12.1994**
(21) Numéro de dépôt: 89913064.5
(22) Date de dépôt: 13.11.1989
(51) Int. Cl.: G01N 33/53, G01N 33/60, G01N 33/68, G01N 33/574

(54) **PROCEDE POUR LE DOSAGE DE LA FORME ACIDE DU FGF DANS LES FLUIDES OU TISSUS BIOLOGIQUES ET SES APPLICATIONS**
VERFAHREN ZUR DOSIERUNG DER SAUREN FORM DES FGF IN FLÜSSIGKEITEN ODER BIOLOGISCHEN GEWEBEN UND SEINE ANWENDUNGEN
PROCESS FOR ASSAYING THE FGF ACID FORM IN FLUIDS OR BIOLOGICAL TISSUES AND APPLICATIONS THEREOF

(30) Priorité: 15.11.1988 FR 8814824
(43) Date de publication de la demande: 04.09.1991
(73) Titulaire: UNIVERSITE PARIS-VAL DE MARNE, F-94010 Créteil Cédex (FR)
(72) Inventeur: BARRITAULT, Denis, Stephen, Charles, F-75001 Paris (FR); CARUELLE, Jean-Pierre, F-94100 St-Maur (FR); CARUELLE LEBRIS, Danièle, Jeanne, F-94100 St-Maur (FR); CHOPIN, Dominique, Khiem, Marie, F-94340 Joinville-le-Pont (FR); VOISIN, Marie-Catherine, Paule, Germaine, F-94300 Vincennes (FR)
(74) Mandataire: Phélip, Bruno
(86) Numéro de dépôt international: FR8900583
(87) Numéro de publication internationale: WO9005915

(56) Documents cités:
- ANALYTICAL BIOCHEMISTRY, vol. 173, 1988, Academic Press Inc.; D. CARUELLE et al., pp. 328-339#
- CHEMICAL ABSTRACTS, vol. 104, no. 15, 14 avril 1986, Columbus, OH (US); A. BAIRD et al., p. 489, no. 127471a#
- CHEMICAL ABSTRACTS, vol. 108, no. 21, 23 mai 1988, Columbus, OH (US); M. WESTPHAL et al., p. 153, no. 180897c#

## Description

La présente invention concerne un procédé pour le dosage de la forme acide des FGF et de ses immuno-équivalents, molécules natives ou tronquées. Elle a pour objet des conditions particulières de dosage immunologique utilisant des anticorps monoclonaux et/ou polyclonaux et des standards correspondant aux formes natives, tronquées, synthétiques et/ou recombinantes des FGF acides dans les fluides ou tissus biologiques. Elle a également pour objet l'application du dosage spécifique de la forme acide des FGF et de ses immunoéquivalents. La quantification de telles molécules, exprimée en immunoréactivité équivalente à FGF, permet le dépistage, le pronostic, le diagnostic, le suivi médical et le bilan d'extension dans le cas de maladies prolifératives ou dégénératives.

La forme acide des FGF et de ses immunoéquivalents constitue selon l'invention, un marqueur biologique caractéristique des situations pathologiques citées ci-dessus et tout particulièrement dans les domaines de la cancérologie et des atteintes inflammatoires.

La découverte de la famille des facteurs de croissance des fibroblastes (couramment désignés par l'abréviation "FGFs - Fibroblast Growth Factor") a été faite à la suite des études des activités biologiques, facteurs de croissance, obtenues dans les extraits d'un très grand nombre de tissus ou d'organes (cerveau, hypophyse, rétine, humeur vitrée, choroïde, iris, cartilage, rein, foie, placenta, corpus luteum, prostate, os, muscle, etc). La diversité même des tissus étudiés et des cellules stimulées par ces facteurs in vitro et in vivo, ainsi que le grand nombre d'équipes de recherche qui ont indépendamment contribué à la caractérisation, à l'isolement et à l'identification complète de ces facteurs, expliquent la multitude des noms et des signes donnés par ces différents auteurs pour les désigner. Il apparaît ainsi maintenant de façon claire que tous ces extraits contiennent des facteurs de croissance de la famille des FGFs et que cette famille peut être représentée par deux branches principales.

La première branche a été décrite sous les noms de FGF basique, de facteur de croissance basique ou de type 2 affin pour l'héparine ou "Heparin Binding Growth Factor" (HBGF II), de facteur de croissance dérivé du cerveau ou "Brain-Derived Growth Factor II" (BDGF II), de facteur de croissance dérivé de l'oeil ou "Eye-Derived Growth Factor I" (EDGF I), de facteur de croissance des astrocytes II ou AGF II, de facteur de croissance dérivé du cartilage (CDGF II) etc., alors que la deuxième branche de la famille FGF a été décrite sous les noms de FGF acide ou facteur affin pour l'héparine de type I (HBGF I), facteur dérivé du cerveau ou BDGF I, de facteur de croissance dérivé de l'oeil ou EDGF II, de facteur de croissance des astrocytes I ou AGF I, de facteur de croissance dérivé du cartilage (CDGF I), etc. A titre de références bibliographiques concernant les facteurs de croissance, on citera Roy R. Lobb, dans "Analytical Biochemistry 154, 1-14 (1986)", et J. Courty et al, dans "Biochem. Biophys. Res. Commun., 136, 102-108 (1986)".

Ces facteurs ont donc été nommés, soit selon le type de cellules cibles utilisées (facteurs de croissance des fibroblastes, astrocytes ou cellules endothéliales, avec les signes respectivement FGF, AGF, ECGF), soit selon la source d'où ce facteur est extrait (par exemple, facteur de croissance dérivé du cerveau, dérivé de la rétine ou des yeux, du cartilage, d'hépatocytes en culture, avec les sigles respectivement BDGF, RDGF, EDGF, CDGF, HDGF, etc.), soit encore selon une propriété biochimique ou biologique [facteurs de croissance affins pour l'héparine (HBGF) ou facteur tumoral angiogénique (TAF)], les deux principales branches de la famille étant alors nommées selon ces sigles précédés ou suivis de la mention "acide" ou "basique, ou de la mention "type I" ou "type II".

C'est en suivant l'activité biologique sur des cellules en culture que ces facteurs ont pu être purifiés. Les premières caractéristiques physico-chimiques (poids moléculaire et point isoélectrique) ont été publiées par Gospodarowicz, J. Biol. Chem. 250, 2515 (1975) pour la forme basique, et par Barritault et coll., J. Neurosci. 8, 477-490 (1982) pour la forme acide. Ainsi, on notera que, selon l'art antérieur, si la forme basique a une répartition tissulaire ubiquitaire, la forme acide a été essentiellement décrite dans les tissus nerveux et dans le rein.

La purification à homogénéité des deux formes du FGF a permis d'établir leurs structures primaires : Esch. F. et coll., Proc. Natl. Acad. Sci. US, 82, 6507 (1985), pour la forme basique, et Gimenez Gallego et coll., Science, 230, 1385-1388 (1985), pour la forme acide. L'isolement des deux formes a été grandement favorisé par la découverte d'une forte affinité de ces facteurs pour l'héparine et l'utilisation subséquente d'une chromatographie d'affinité sur héparine immobilisée [Shing et coll., Science, 223, 1296-1299 (1984)].

In vitro, les FGFs sont capables de stimuler la prolifération et la différenciation d'un grand nombre de cellules provenant de tissus et espèces différents. On citera les cellules fibroblastiques, endothéliales, épithéliales, les kératinocytes, chondrocytes, myoblastes, astrocytes, etc. On signalera également le rôle des FGFs dans la survie neuronale. In vitro, les propriétés neurotrophiques, angiogéniques et cicatrisantes des FGFs ont également été démontrées dans de nombreux systèmes.

Les connaissances des effets in vivo sont relativement restreintes. Les FGFs et particulièrement la forme basique ont été rapportés comme des molécules intervenant dans le développement embryonnaire, dans les mécanismes de la régénération des pattes et du cristallin chez les amphibiens. Ces molécules sont envisagées comme des agents neurotrophiques et impliquées dans les mécanismes de réparation tissulaire. La dernière propriété qui concerne l'effet angiogénique in vivo représente une propriété importante dans les mécanismes de l'embryogenèse et dans la progression des cellules tumorales.

Un des grands intérêts de l'étude des facteurs angiogéniques est la relation qui existe entre la progression de tissus tumoraux et les mécanismes de neoangiogenèse associés.

La plupart des observations réalisées à propos des relations entre les FGFs et les pathologies concerne la forme basique de cette famille de facteurs. La mise au point d'un dosage immunologique spécifique de la forme acide [Caruelle et Coll. Analytical Biochemistry, 173, 001-0012, (1988)] a permis de mettre en évidence la présence de la forme acide dans des fluides et des tissus biologiques jusqu'alors considérés comme des territoires où seule la forme basique a été décrite et où la forme acide est considérée comme absente ou présente en quantité très faible. Ainsi la présence de FGF acide ou d'immuno-équivalents désignés ci-après par l'abréviation aFGF n'a à ce jour et à la connaissance du demandeur, jamais été décrite chez l'homme ou d'autres espèces animales, dans les tissus comme le cartilage, dans la prostate, la vessie etc. ou dans les fluides : sang, urine, alors que la forme bFGF a été décrite.

La présente invention met pour la première fois en évidence la présence de aFGF dans certains tissus, décrit les conditions spécifiques du dosage du aFGF et de ses immunoéquivalents adaptés à des extraits tissulaires et des fluides biologiques et tire profit d'un tel dosage à la reconnaissance de certains états pathologiques, particulièrement dans le domaine de la cancérologie.

L'invention concerne un procédé de dosage de la forme acide des FGF et de ses immunoéquivalents (aFGF) dans les fluides et tissus biologiques, dans lequel on utilise un traceur conjugué à ladite forme acide et pouvant être reconnu par des anticorps spécifiques de cette même forme, caractérisé en ce que le dosage est effectué à une force ionique au moins égale à 0,7 M NaCl, permettant la dissociation des formes complexées de aFGF présentes dans lesdits fluides et tissus biologiques.

L'invention a encore pour objet un procédé de reconnaissance d'états pathologiques, notamment dus à des maladies prolifératives, dégénératives ou inflammatoires, selon lequel on effectue sur un échantillon de fluide ou tissu biologique le dosage de la forme aFGF qu'il peut contenir, la quantité de aFGF ainsi déterminée constituant une valeur représentative dudit état pathologique.

Les facteurs de croissance sont des polypeptides qui peuvent stimuler ou inhiber la croissance cellulaire grâce à une liaison spécifique de haute affinité avec des récepteurs cellulaires. Les relations étroites entre les facteurs de croissance et le produit de certains oncogènes, leurs modes d'action, leurs degrés d'expression, leurs récepteurs font apparaître ces molécules comme des agents responsables de l'homéostasie cellulaire.

Pourtant, jusqu'à la présente invention, il n'avait jamais, à la connaissance du demandeur, été proposé ni suggéré d'utiliser la quantité d'aFGF éventuellement présente dans le fluide ou tissu biologique étudié, comme valeur représentative de l'état pathologique.

Les marqueurs tumoraux représentent une aide considérable sur le plan de l'aide au diagnostic et à la thérapeutique. Dans le cadre des tumeurs uro-génitales de l'homme, en dehors des tumeurs de la prostate et du testicule, on ne connait pas, dans la technique antérieure, de marqueurs utilisés en pratique courante.

Selon l'invention, on a trouvé que la forme acide du FGF est un marqueur tumoral ou un marqueur d'inflammation, en raison de sa teneur naturellement nulle ou très faible dans les tissus en situation physiologique normale et de ses variations dans différentes pathologies.

On préfère un dosage immunoenzymatique, mais on peut également faire appel à un dosage radioactif à l'aide d'un marqueur isotopique, tel que le 125 iode, le tritium, le 14 carbone, le 35 soufre par exemple). De même, tous systèmes d'amplification comme les systèmes avidine-biotine peuvent être mis en oeuvre dans le dosage.

On peut aussi utiliser des techniques de marquage avec des composés luminescents, notamment fluorescents, ou magnétiques.

D'une manière générale, tous types de dosages immunologiques, dosages par réaction à l'équilibre ou dosages immunométriques, en particulier la technique Sandwich et ses dérivés utilisent les anticorps spécifiques ou des fragments d'anticorps couplés à un traceur de quelque nature que ce soit applicables.

Certains aspects du dosage immunoenzymatique à l'ACHE (acétylcholine-estérase) sont décrits dans la publication précitée de Caruelle et Coll. et l'homme du métier peut s'y référer, en cas de besoin.

La présente invention concerne des conditions de dosage spécifiquement adaptées aux tissus et fluides.

Les plaques de microtitration préalablement traitées avec le deuxième anticorps (anti-Fc de lapin ou de souris par exemple, choisis selon l'espèce animale d'origine de l'immunsérum spécifique) sont saturées avec du tampon phosphate, 100 mM phosphate, 0,4 M NaCl, 10-3 M EDTA, 1 % d'albumine sérique et 0,01 % NaN3 (tampon ultérieurement appelé PBS + 0,4 M NaCl). Les plaques sont ensuites conservées à 4°C avant utilisation.

Le volume réactionnel d'un volume final de 150 microlitres se décompose en :
- 50 microlitres d'une solution standard de concentration définie de aFGF dans un tampon phosphate salin de force ionique au moins égale à 0,7 M NaCl pour l'établissement de la courbe étalon ou 50 microlitres de la solution à doser,
- 50 microlitres d'une solution dans le même tampon de conjugé enzymatique aFGF-ACHE à une unité Elmann par millilitre,
- 50 microlitres de sérum anti-aFGF dans le même tampon à la dilution de travail.

Après une nuit d'incubation à 22°C, la réaction antigène-anticorps est arrivée à l'équilibre et les plaques sont lavées. 200 microlitres de réactif d'Elmann sont déposées dans chaque puits. Après un temps de réaction compris entre 30 minutes et deux heures, la densité optique de chaque puits est mesurée par un spectrocolorimètre à 414 nanomètres.

Les liaisons non spécifiques sont établies en réalisant des réactions où l'anti-aFGF est remplacé par 50 microlitres de tampon PBS et sont appelées T. La liaison maximum du traceur sur l'immunsérum est définie en remplaçant les 50 microlitres de la solution standard de aFGF par 50 microlitres de tampon PBS ; elle correspond au BO.

A chaque point expérimental (gamme ou échantillons à doser) la densité optique correspond à une valeur appelée B.

Les résultats sont exprimés sous la forme de B-T/BO-T en %.

Chaque dosage est réalisé au moins en duplicate.

Selon ce protocole et en fonction de la force ionique finale du milieu réactionnel, ce dosage se caractérise par une sensibilité (B-T/BO-T = 50 %) d'environ 2 nanogrammes de aFGF immunoéquivalent par millilitre pour une force ionique finale de 1 M NaCl.

Les limites de détection (définies pour B-T/BO-T = 80 %) sont d'environ 700 picogrammes de aFGF immunoéquivalent par millilitre.

Un des objets de l'invention est la réalisation de ce dosage dans un tampon de force ionique finale au moins égale à 0,7 M NaCl et dans des conditions telles que la reconnaissance antigène-anticorps puisse s'effectuer. On préfère une force ionique réactionnelle de l'ordre de 1 M NaCl.

Cette concentration saline constitue un milieu dissociant pour les complexes moléculaires associant et/ou protégeant le aFGF, avec d'éventuels transporteurs ou complexants de type héparines, proteo héparines, glycosaminoglycane, etc...

La validité du dosage immunoenzymatique à cette force ionique par exemple d'environ 1 M NaCl permet donc de dissocier le aFGF naturellement complexé dans des conditions de forces ioniques plus faibles (de 0 M NaCl à O,7 M NaCl) et donc de quantifier ces molécules démasquées immunologiquement.

Ce dosage est applicable à toutes formes de fluides ou tissus biologiques :
- des fluides biologiques comme le sang, le sérum, les urines, le liquide céphalorachidien, des exsudats, etc...
- des tissus de toutes natures, durs (os, cartilage), mous (viscères, peau, etc...).

A partir des liquides et fluides biologiques, les doages sont effectués après dilution, volume à volume, de ces extraits avec du PBS 2 M.

Pour ce qui concerne les tissus, il est nécessaire de les broyer dans du tampon PBS 2 M et de ramener la force ionique à environ 1 M NaCl pour dosage.

Les spécificités zoologiques de la forme acide du FGF n'interfèrent pas dans les capacités de reconnaissance de l'immunsérum. Cette forme moléculaire est très conservée à travers les différentes espèces animales. L'anticorps obtenu par immunisation avec du FGF acide d'origine bovine présente une réactivité croisée de 100 % avec le FGF acide humain, murin, avien, sauropsidéen, de batracien et de poissons.

Ce dosage est donc exploitable à partir d'échantillons provenant de toutes les espèces animales et en particulier de toutes les classes de Cordés.

Le dosage du aFGF dans les fluides biologiques ou à partir de biopsies peut être utilisé tout particulièrement :
- chez les patients ayant des tumeurs connues pour préciser le bilan d'extension qui comporte actuellement une erreur pouvant aller jusqu'à 40 % avec les marqueurs de diagnostic et d'imagerie actuelle ;
- chez les patients ayant des masses tumorales apparemment localisées où le taux de aFGF peut avoir une valeur pronostique justifiant un traitement complémentaire systémique après traitement locorégional ;
- chez les patients ayant subi une résection de masse tumorale pour apprécier la persistance ou la recrudescende d'éléments tumoraux non décelables ;
- pour évaluer le critère d'agressivité de masses tumorales superficielles ou non, à l'état quiescent.

Egalement pour des affections fréquentes, ce marqueur peut avoir un intérêt de diagnostic dans le cadre de dépistage.

L'invention présente notamment un grand intérêt dans les états pathologiques ci-après indiqués à titre d'exemples illustratifs et non limitatifs.

Les tumeurs du rein se caractérisent par leur latence et le fait que, dès que la tumeur a dépassé le premier relais ganglionnaire, aucune thérapeutique ne peut modifier radicalement la survie. La seule arme thérapeutique efficace reste la chirurgie, à condition que la tumeur soit diagnostiquée à temps. Toutefois, de nombreux essais utilisant les modificateurs de la réponse biologique (Interférons, interleukine 2) et l'immunothérapie (lymphocytes activés LAK, TIL) semblent démontrer qu'il est possible d'obtenir chez certains patients une stabilisation, voire une régression, du processus métastatique. Ces résultats indiquent qu'il est possible de contrôler la croissance de certaines métastases de cancer du rein, notamment en cas de lésions pulmonaires exclusives. La possibilité de disposer d'un marqueur tumoral représente une aide importante au diagnostic, au pronostic et à la thérapeutique.

Les tumeurs de la vessie représentent un large spectre de différents processus qui sont ou non interdépendants. Les données épidémiologiques, au contraire des données expérimentales et des constatations histologiques, ont démontré que plusieurs schémas étaient possibles.
* Les tumeurs superficielles de la vessie (absence d'infiltration musculaire) peuvent soit récidiver mais évoluer sur un mode superficiel, soit rapidement ou à un moment donné de leur évolution devenir infiltrantes (de 4 à 30 % des cas en fonction de critères pronostiques). Tout se passe comme si une deuxième population cellulaire ayant les propriétés permettant d'infiltrer la paroi vésicale et de disséminer à l'ensemble de l'organisme était mise en jeu. Les dysplasies et le carcinome in situ (CIS) peuvent être associés à tous les types de proliférations urothéliales, suggérant le caractère diffus ou plurifocal du processus transformant au sein de l'épithélium urinaire. Les dysplasies et le CIS peuvent être isolés et bien réagir au traitement conservateur (instillations endovésicales), alors que d'autres, en l'absence de traitement radical précoce, ont un potentiel métastatique (leur association à une tumeur vésicale est de mauvais pronostic).
* Les tumeurs infiltrantes (infiltration musculaire) sont une affection gravissime. Sur les séries historiques de patients non traités, la survie à un an est inférieure à 5 % avec la moitié des patients décédés dans les six mois. D'un point de vue pratique, le problème est de savoir si la tumeur a déjà métastasé au moment du diagnostic, et peut bénéficier d'un traitement radical locorégional. Le risque d'erreur dans le bilan d'extension de ces tumeurs malgré l'imagerie moderne peut aller jusqu'à 40 %. Il importe donc de définir des critères pronostiques d'agressivité, car il semble bien que certaines tumeurs aient un potentiel métastatique précoce, et qu'il existe au moment du diagnostic des micrométastases non décelables actuellement. Pour une tumeur invasive apparemment limitée à la vessie, le risque de décès par métastase dans les deux ans est évalué à 50 %. Il apparaît donc important de mieux stratifier les tumeurs infiltrantes de la vessie afin de mieux cerner celles qui vont bénéficier d'un traitement locorégional.
* Les tumeurs se présentant avec des métastases d'emblée, voient dans certains cas leur évolution modifiée par l'association d'agents chimiothérapiques. Toutefois, chez les patients répondeurs, il semble bien que l'on ne puisse espérer qu'une amélioration transitoire des courbes de survie. La venue d'agents thérapeutiques nouveaux est donc dans ce domaine d'une nécessité absolue.

Dans tous les cas de tumeurs de la vessie, l'invention procure un moyen d'investigation non invasif beaucoup moins coûteux que l'endoscopie. En outre, le dosage des aFGF peut être pratiqué sur l'urine brute, ce qui représente une grande simplification.

Les tumeurs de la prostate se caractérisent par leur fréquence (deuxième cause de décès par cancer chez l'homme actuellement) et leur hormonodépendance. Il est actuellement en France le plus souvent diagnostiqué au stade métastatique. Le dépistage par le toucher rectal, l'échographie et les marqueurs tumoraux pourraient permettre de modifier cet état de fait. Au stade métastasé, le traitement repose sur la suppression androgénique. Quelles que soient les modalités du traitement hormonal, on observe une réponse dans 80 à 90 % des cas, avec un échappement hormonal dans un délai moyen de dix-huit mois dans la majorité des cas. La prolifération tumorale n'est alors que médiocrement contrôlée par la chimiothérapie, avec un taux de réponse pouvant atteindre 35 % des cas. Il apparaî+ indispensable de mieux comprendre et de définir la population hormonoindépendante au sein de la tumeur prostatique, ses mécanismes de contrôle et de mise en jeu.

L'invention concerne aussi un coffret (ou kit) en vue de l'application du dosage des aFGF, comprenant :
- un traceur résultant du couplage du aFGF avec un marqueur,
- un compétiteur consistant en du aFGF natif, tronqué, synthétique ou recombinant sous la forme d'une solution standard de aFGF de concentration définie pour l'établissement d'une courbe-étalon,
- des anticorps polyclonaux et/ou monoclonaux reconnaissant spécifiquement l'aFGF.

En variante, l'invention concerne aussi un coffret comprenant :
- un traceur résultant du couplage avec un marqueur des anticorps polyclonaux et/ou monoclonaux reconnaissant spécifiquement l'aFGF,
- des anticorps polyclonaux et/ou monoclonaux, non marqués, reconnaissant spécifiquement l'aFGF,
- une solution standard de aFGF de concentration définie pour l'établissement de la courbe-étalon.

Les réactifs sont présentés pour réaliser le dosage en phase liquide ou en phase solide.

Diverses formes d'immunoadsorbants peuvent être utilisées pour les anticorps et les antigènes, par exemple des billes ou des supports en nitrocellulose (bandelettes).

Les observations rapportées dans les exemples détaillés ci-après ont été effectuées sur des biopsies humaines de tissus sains et tumoraux, ainsi que sur des urines et sur du sérum.

Les exemples développés ci-dessous, lequels concernent plus de deux cents dosages et observations, traduisent d'une part la présence de la forme acide dans des prélèvements tumoraux (chondrosarcomes, tumeurs de l'appareil urogénital) et surtout la variation de la teneur en cette molécule en fonction des degrés de la maladie considérée. La variation de cette teneur entre les tissus témoins et les tissus pathologiques permet de préciser le pronostic, le diagnostic, le suivi médical, le bilan d'extension et le dépistage de pathologies néoplasiques ou inflammatoires.

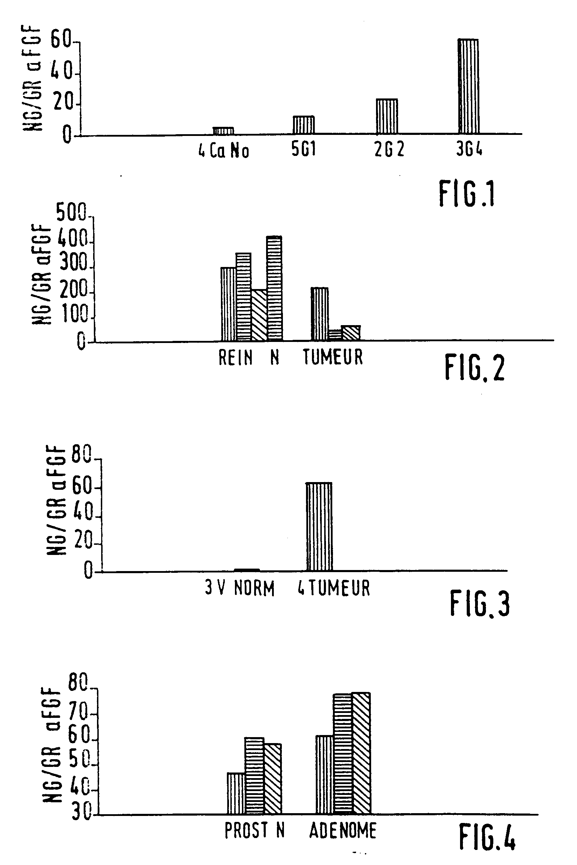

Les résultats sont illustrés par les Figures 1 à 13 annexées ; les légendes de ces Figures respectives sont reprises en fin de description.

### EXEMPLE 1

Estimation de la teneur en aFGF dans des cartilages humains sains et dans des chondrosarcomes

La teneur en aFGF a été recherchée dans des biopsies de chondrosarcomes (tumeurs de cartilage) de degré évolutif croissant en comparaison avec des cartilages sains de référence.

Les prélèvements de cartilage considérés comme des cartilages de référence ont été obtenus à partir de tête de fémur, d'os iliaque, de sternum.

Ces différents prélèvements, conservés à - 80° C ont été pulvérisés dans de l'azote liquide et broyés dans un milieu physiologique tamponné de haute force ionique en sel. Ce tampon est du tampon phosphate 100 mM phosphate, pH 7,4 + 2 M NaCl, appelé ultérieurement PBS 2 M. Toutes les opérations se déroulent dans la glace fondante ou à 4° C.

Après centrifugation des broyats, les surnageants de centrifugation sont récupérés et leur force ionique est amenée à une force ionique de 0,65 M NaCl par dilution avèc du tampon phosphate 10 mM pH 7,4.

Une chromatographie d'affinité sur gel d'héparine Sépharose (Pharmacia) permet ensuite de fixer la forme acide du FGF et de le désorber par une élution à une force ionique de 1 M NaCl. Les dosages sont effectués dans cet éluat par rapport à une courbe étalon réalisée dans un tampon identique, c'est à dire de même force ionique que celle des tampons d'élution de la colonne.

Ainsi qu'on l'a mentionné ci-dessus, le dosage du aFGF selon Caruelle et Coll. (1988) consiste en un dosage à l'équilibre par compétition d'accrochage des antigènes recherchés vis à vis d'un traceur obtenu par greffage de la forme aFGF avec une enzyme, l'acétylcholine-estérase. Ce dosage est réalisé dans des plaques de microtitration de 96 puits. L'activité enzymatique révélée par le réactif d'Elmann permet de quantifier le compétiteur immunologiquement équivalent au aFGF en se rapportant à une courbe étalon réalisée sur la même plaque et dans les mêmes conditions de force ionique du milieu réactionnel.

Les dosages peuvent ainsi être effectués sur le surnageant de broyage. La teneur en aFGF de l'élution du gel d'héparine sépharose par du PBS 1 M NaCl est estimée par rapport à une gamme réalisée dans un tampon de même force ionique.

La Figure 1 représente la teneur en aFGF immuno-équivalent (aFGF i.é.) établie à partir de cartilages témoins ou de chondrosarcomes de stades évolutifs croissants. Les valeurs représentent la moyenne des expériences réalisées indépendamment. Elles sont exprimées en nanogramme de aFGF i.é. par gramme de poids frais de la biopsie.

Les résultats obtenus montrent qu'il existe une corrélation entre la teneur en aFGF i.é. et le degré de la maladie. Plus les tumeurs ont un caractère agressif ou plus les cellules sont dans un état indifférencié, plus la teneur en aFGF y est élevée.

Le dosage de aFGF permet donc de préciser le diagnostic de niveau de malignité établi essentiellement sur des critères histologiques.

### EXEMPLE 2

Quantification de l'immunoréactivé aFGF dans des biopsies de l'appareil urogénital de patients sains ou atteints d'affections bégnines ou malignes.

Des prélèvements de différents organes de l'appareil urogénital de l'homme ont été utilisés pour l'établissement de la teneur en aFGF.

Ces biopsies de tissus sains ou correspondant à des situations pathologiques définies par des critères histologiques sont conservées à - 80° C avant utilisation. Le protocole d'extraction des FGF acide est équivalent à celui présenté dans l'exemple 1.

Les résultats de ces expériences sont présentés sur les Figures 2, 3 et 4.

Les Figures 2 et 3 rapportent les teneurs en aFGF i.é. respectivement dans des biopsies de reins sains et tumoraux et dans des vessies saines et des tumeurs de l'urothelium vésical.

Les dosages réalisés sur ces deux types d'organes font apparaître des teneurs différentes entre les organes sains et les organes pathologiques.

Dans le cas des reins, l'état néoplasique se traduit par une diminution du taux endogène de aFGF.

Dans le cas des affections de la vessie, la teneur en aFGF est plus élevée dans les tissus tumoraux que dans les organes témoins.

Ces observations ont été confirmées par des études immunohistologiques.

La Figure 4 rapporte les variations de la teneur en aFGF i.é. dans des adénomes de prostate comparées à celle de prostate saine. Les adénomes révèlent une immuno-réactivité plus importante que celle détectée dans les organes témoins.

Le dosage de aFGF peut donc être utilisé dans des biopsies cancéreuses comme un moyen de dépistage, de diagnostic ou de suivi de maladies des tumeurs ou affections des organes de l'appareil urogénital.

### EXEMPLE 3

Estimation de l'immunoréactivité de type aFGF dans les urines.

La teneur en aFGF a été estimée dans les urines d'une population de patients atteints de pathologies tumorales et inflammatoires. La population témoin est la population d'individus ne présentant pas ce type d'affections.

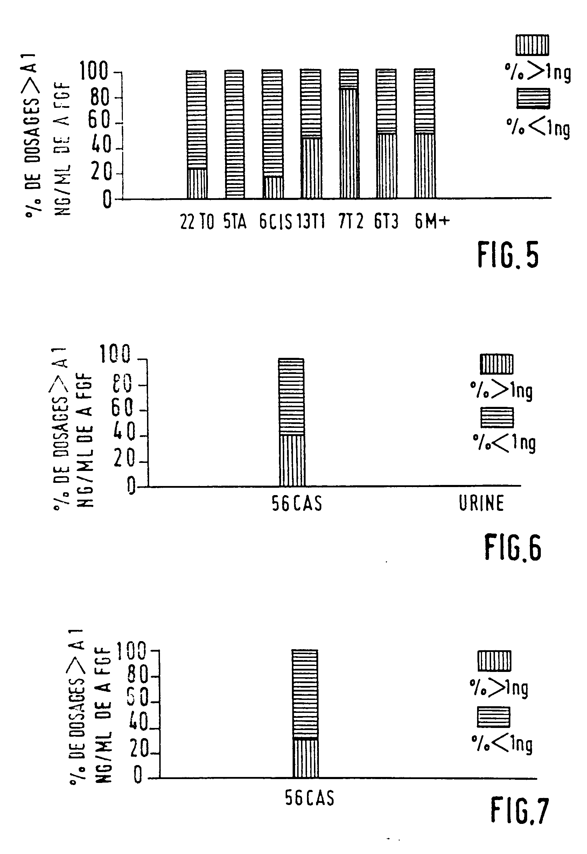

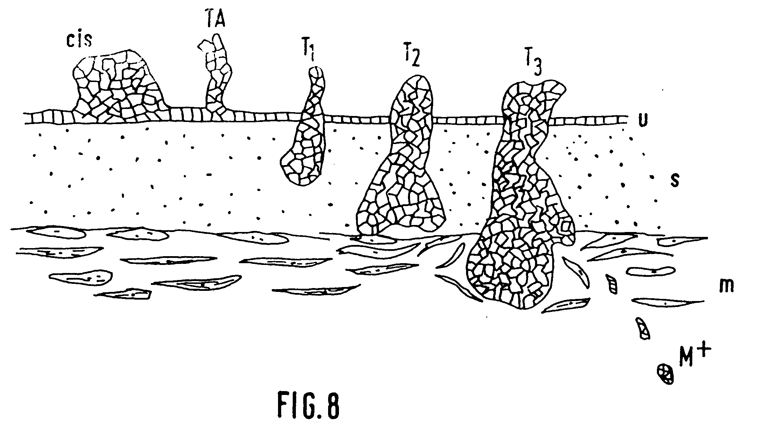

Les Figures 5, 6 et 7 présentent les immuno-réactivités d'une valeur au moins égale à 1 nanogramme par millilitre d'urine détectée dans des urines de patients atteints de différentes maladies. La Figure 5 rapporte le pourcentage d'urines considérées comme positives selon ce critère (c'est à dire au moins 1 nanogramme de aFGF i.é. par millilitre d'urine) pour une population d'individus présentant une prolifération superficielle de l'urothelium (TA, CIS), une affection maligne de degré croissant (T1, T2, T3), des métastases d'origine vésicales (M+) ou des patients ayant subi une résection de la vessie (TO) et n'ayant pas de tumeurs décelables par les marqueurs conventionnels d'imagerie actuellement disponibles. Ces différentes catégories correspondent à des positions et à des degrés d'invasion des masses tumorales présentées schématiquement Figure 8.

La Figure 6 présente le pourcentage d'immuno-réactivité positive dans le cas de cancer de prostate.

La Figure 7 présente le pourcentage d'immuno-réactivité d'urines prélevées sur des individus atteints d'adénomes de prostate.

La Figure 9 présente les résultats obtenus à partir de dosages réalisés sur une population considérée comme une population témoin dans laquelle deux groupes ont été considérés : les témoins ne présentant pas de situation inflammatoire (-), ceux présentant une pathologie inflammatoire, de type calcul rénal, notés (+).

Il ressort de ces observations que ce test permet, selon les populations considérées de détecter à partir d'un dosage dans les urines, donc d'une manipulation non traumatique, un pourcentage significatif (35 % environ) d'individus présentant les affections sélectionnées dans cette étude.

Ce dosage se présente donc comme un outil fiable de dépistage compte tenu de l'absence de marqueurs tumoraux à large spectre dans les urines.

Il constitue aussi un critère de diagnostic très important pour le suivi des malades en situation post opératoire. Il peut permettre dans le cas des proliférations superficielles et des tumeurs reséquées (TO) de préciser le potentiel agressif de masses tumorales apparemment inactives.

Ce dosage peut enfin être utilisé comme un outil d'information en réponse au traitement des pathologies.

Ainsi en plus de l'utilisation de ce dosage à titre de dépistage, l'estimation de l'immunoréactivité aFGF et le suivi de ses variations au cours du temps peuvent être considérés comme des informations permettant de prévenir ou de suivre l'évolution d'un malade.

### EXEMPLE 4

Estimation de l'immunoréactivité de type aFGF dans le sérum.

La teneur en immunoréactivé dans le sérum de patients atteints de pathologies variées a été établie avec le même type de dosage.

Jusqu'alors aucune information concernant les formes circulantes de ces molécules n'a été publiée.

En réalisant le dosage dans un tampon à haute force ionique (environ 1 M NaCl), c'est à dire dans un milieu dissociant, une immunoréactivité de type aFGF a pu être enregistrée dans les sérums de certains malades. Cette force ionique élevée démasque une immunoréactivité non détectable dans des conditions normales de dosage c'est à dire avec une force ionique de l'ordre de 0,4 M NaCl.

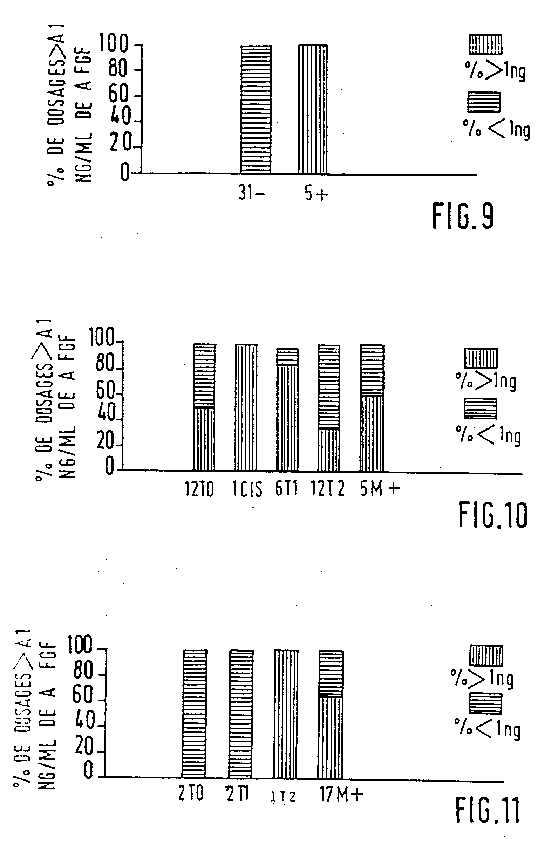

La Figure 10 présente le pourcentage de sérums jugés immunopositifs (c'est à dire contenant au moins 1 nanogramme par millilitre) par populations d'invidus présentant une pathologie définie de type cancer de vessie.

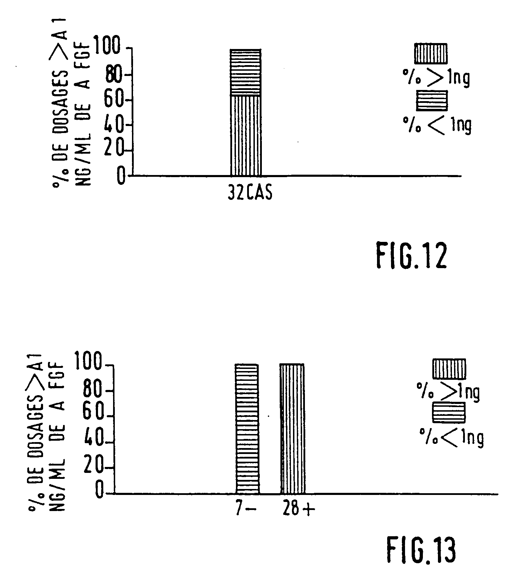

La Figure 11 présente les mêmes informations pour des pathologies du type cancer de prostate, la Figure 12 concerne des adénomes de prostate.

La Figure 13 rapporte le pourcentage d'immuno-réactivité d'une valeur supérieure à 1 nanogramme par millilitre dans une population de témoins subdivisés en deux groupes. Le groupe noté (-) ne présente pas de situations inflammatoires, le groupe noté (+) présente des situations inflammatoires.

Il ressort de ces résultats que la détection dans le sérum d'une immunoréactivité vis à vis du FGF acide peut être mise en relation avec une situation physiologique signalétique d'une prolifération cellulaire bénigne ou maligne.

### EXEMPLE 5

Evaluation de la teneur en aFGF dans les zones tumorales et infiltrées de tumeurs gliales hémisphériques chez l'homme adulte

Le dosage du aFGF a été réalisé dans les mêmes conditions dissociantes (1 M NaCl) que dans l'exemple 4 sur des biopsies de tumeurs gliales, prélevées directement dans le bloc opératoire et conservées instantanément dans l'azote liquide.

Ces dosages sont réalisés sur des prélèvements correspondant d'une part à la zone tumorale sensu stricto et d'autre part aux régions périphériques infiltrées, richement néovascularisées, mais d'aspect macroscopique non tumoral. Les échantillons témoins sont prélevés dans des conditions post mortem répondant aux mêmes préoccupations opératoires. Chacun de ces prélèvements est soumis parallèlement à une analyse histologique et à un dénombrement de la population de cellules tumorales et endothéliales par coloration spécifique exploitée par analyse d'image.

Les résultats concernant le dosage du aFGF montrent que pour des biopsies de tissus sains, deux régions adjacentes présentent un taux identique de facteur (ratio moyen de 1,009 ± 0,019 pour 4 cas).

Dans le cas des tumeurs de faible agressivité, le rapport de la teneur en aFGF entre la zone infiltrée et la zone tumorale est toujours supérieur à 1. La moyenne établie sur 4 cas est de 1,491 = ± 0,185. Ce rapport dans le cas des tumeurs malignes est toujours supérieur à 2 pour 6 cas étudiés (m = 3,960 ± 1,959). En valeur absolue, ce rapport est d'autant plus élevé que le grade histologique est plus fort. L'augmentation du taux de FGF acides corrèle également les critères histologiques se rapportant aux dénombrement des cellules endothéliales.

FIGURE 1 :

Teneur en aFGF i.é. exprimée en nanogramme par gramme de poids frais dans des biopsies de cartilage témoin (4 cas : Ca NO), dans des tumeurs bénignes (5 cas : 5 G1) ou dans des tumeurs malignes d'agressivité croissante (2 cas : G2 et 3 cas : G4). Les histogrammes présentent la valeur moyenne des expériences menées indépendamment.

FIGURE 2 :

Teneur en aFGF i.é. exprimée en nanogramme par gramme de poids frais dans des biopsies de rein normal (REIN N) et dans des tumeurs de rein (TUMEUR). Les histogrammes présentent la valeur moyenne des expériences menées indépendamment.

FIGURE 3 :

Teneur en aFGF i. é. exprimée en nanogramme par gramme de poids frais dans des biopsies de vessie normale (3 cas : 3 V NORM) et dans des tumeurs de vessie (4 cas : 4 TUMEURS). Les histogrammes présentent la valeur moyenne des expériences menées indépendamment.

FIGURE 4 :

Teneur en aFGF i.é. exprimée en nanogramme par gramme de poids frais dans des biopsies de prostate témoin (3 cas : PROST N) et dans des adenomes prostatiques (3 cas : ADENOME). Les histogrammes présentent la valeur moyenne des expériences menées indépendamment.

FIGURE 5 :

Histogrammes présentant le pourcentage d'immuno-réactivités d'une valeur supérieure à 1 nanogramme par millilitre (en sombre) détectées dans les urines d'un groupe de malades atteints de différentes pathologies de cancer de vessie.
22 TO : résection de la vessie
Tumeurs superficielles :
5 TA, 6 CIS, 13 T1.
Tumeurs invasives de degré croissant :
7 T2, 6 T3, 6 M plus.

FIGURE 6 :

Histogrammes présentant le pourcentage d'immuno-réactivités d'une valeur supérieure à 1 nanogramme par millimitre (en sombre) détectées dans les urines d'un groupe de malades atteints de différentes pathologies de cancer de prostate (56 cas considérés).

FIGURE 7 :

Histogramme présentant le pourcentage d'immuno-réactivités d'une valeur supérieure à 1 nanogramme par millilitre (en sombre) détectées dans les urines d'un groupe de malades atteints d'adénomes prostatiques (56 cas considérés).

FIGURE 8 :

Schéma présentant les différentes formes de proliférations malignes issues de la vessie. CIS (carcinome in situ, TA, T1) : proliférations superficielles.
T2, T3, M plus : tumeurs invasives.
U : urothélium de la vessie
S : stroma sous-jacent
m : musculaire.

FIGURE 9 :

Histogrammes présentant le pourcentage d'immuno-réactivités d'une valeur supérieure à 1 nanogramme par millilitre (en sombre) détectées dans les urines d'un groupe de témoin sans situation inflammatoire et le pourcentage d'immunoréactivités d'une valeur inférieure à 1 nanogramme par millilitre (en clair) détectées dans les urines d'un groupe de témoins avec une situation inflammatoire (exemple: calcul rénal).

FIGURE 10 :

Histogrammes présentant le pourcentage d'immuno-réactivités d'une valeur supérieure à 1 nanogramme par millilitre (en sombre) détectées dans le sérum d'un groupe de malades atteints de cancer de vessie. Les sous-groupes sont identiques à ceux présentés dans la Figure 5.

FIGURE 11 :

Histogrammes présentant le pourcentage d'immuno-réactivités d'une valeur supérieure à 1 nanogramme par millilitre (en sombre) détectées dans le sérum d'un groupe de malades atteints de cancer de la prostate. Les résultats sont présentés en fonction des types de tumeurs.

FIGURE 12 :

Histogramme présentant le pourcentage d'immuno-réactivités d'une valeur supérieure à 1 nanogramme par millilitre (en sombre) détectées dans le sérum d'un groupe de malade atteints d'adénome de prostate.

FIGURE 13 :

Histogrammes présentant le pourcentage d'immuno-réactivités d'une valeur supérieure à 1 nanogramme par millilitre (en sombre) détectées dans le sérum d'un groupe de témoins sans situation inflammatoire (7 cas) et le pourcentage d'immunoréactivités d'une valeur inférieure à 1 nanogramme par millilitre (en clair) détectées dans le sérum d'un groupe de témoins avec une situation inflammatoire (28 cas).

## Revendications

1. Procédé de dosage de la forme acide des FGF aFGF, acidic fibroblast growth factor et de ses immunoéquivalents dans les fluides et tissus biologiques, dans lequel on utilise un traceur conjugué à ladite forme acide et pouvant être reconnu par des anticorps spécifiques de cette même forme, caractérisé en ce que le dosage est effectué à une force ionique au moins égale à 0,7 M NaCl, permettant la dissociation des formes complexées de aFGF présentes dans lesdits fluides et tissus biologiques.

2. Procédé selon la revendication 1, caractérisé en ce qu'on utilise une technique de dosage immunoenzymatique.

3. Procédé selon l'une des revendications 1 ou 2, caractérisé en ce que le traceur résulte du couplage de aFGF avec un marqueur enzymatique, tel que l'acétylcholine-estérase.

4. Procédé selon la revendication 1, caractérisé en ce qu'on utilise un marqueur radioactif, luminescent ou magnétique.

5. Procédé selon l'une quelconque des revendications 1 à 4, caractérisé en ce qu'on utilise un traceur résultant du couplage avec les formes natives, tronquées, synthétiques ou recombinantes du aFGF.

6. Procédé selon l'une quelconque des revendications 1 à 5, caractérisé en ce qu'on utilise des anticorps polyclonaux et/ou monoclonaux reconnaissant spécifiquement les aFGF.

7. Procédé selon l'une quelconque des revendications 1 à 6, caractérisé en ce qu'on effectue le dosage à une force ionique voisine de 1 M NaCl.

8. Procédé selon l'une quelconque des revendications 1 à 7, caractérisé en ce que l'anticorps est lui-même conjugué, en étant alors utilisé comme marqueur, auquel cas le aFGF est lui-même marqué ou non.

9. Utilisation du aFGF comme marqueur biologique caractéristique d'états pathologiques, en particulier dans les domaines de la cancérologie, des atteintes inflammatoires et dégénératives.

10. Procédé de reconnaissance d'états pathologiques notamment dus à des maladies prolifératives, dégénératives ou inflammatoires, selon lequel on effectue sur un échantillon de fluide ou tissu biologique le dosage de la forme aFGF qu'il peut contenir, la quantité àe aFGF ainsi déterminée constituant une valeur représentative dudit état pathologique.

11. Coffret de réactifs pour la mise en oeuvre du procédé selon l'une quelconque des revendications 1 à 8, ou pour l'application selon la revendication 9, ledit coffret comprenant :
- un traceur résultant du couplage du aFGF avec un marqueur,
- un compétiteur consistant en du aFGF natif, tronqué, synthétique ou recombinant sous la forme d'une solution standard de aFGF de concentration définie pour l'établissement d'une courbe étalon,
- des anticorps polyclonaux et/ou monoclonaux reconnaissant spécifiquement l'aFGF.

12. Coffret de réactifs pour la mise en oeuvre du procédé selon l'une quelconque des revendications 1 à 8, ou pour l'application selon la revendication 9, ledit coffret comprenant :
- un traceur résultant du couplage avec un marqueur des anticorps polyclonaux et/ou monoclonaux reconnaissant spécifiquement l'aFGF,
- des anticorps polyclonaux et/ou monoclonaux, non marqués, reconnaissant spécifiquement l'aFGF,
- une solution standard de aFGF de concentration définie pour l'établissement de la courbe-étalon.

13. Coffret selon l'une des revendications 11 ou 12, caractérisé en ce que les réactifs sont présentés pour réaliser le dosage en phase liquide ou solide.

## Patentansprüche

1. Verfahren zur Bestimmung der Säureform von FGF (aFGF, acidic fibroblast growth factor) und seiner Immunäquivalente in biologischen Flüssigkeiten und Geweben, bei dem man einen mit der Säureform verbundenen Tracer verwendet, der von spezifischen Antikörpern derselben Form erkannt werden kann, dadurch gekennzeichnet, daß die Bestimmung bei einer Ionenstärke von wenigstens gleich 0.7 M NaCl durchgeführt wird, die die Dissoziation der in den biologischen Flüssigkeiten und Geweben komplexierten Formen des aFGF ermöglicht.

2. Verfahren nach Anspruch 1, dadurch gekennzeichnet, daß man eine immunenzymatische Bestimmungstechnik verwendet.

3. Verfahren nach einem der Ansprüche 1 oder 2, dadurch gekennzeichnet, daß der Tracer aus der Kupplung des aFGF mit einem enzymatischen Marker wie Acetylcholin-Esterase hervorgeht.

4. Verfahren nach Anspruch 1, dadurch gekennzeichnet, daß man einen radioaktiven, lumineszierenden oder magnetischen Marker verwendet.

5. Verfahren nach einem der Ansprüche 1 bis 4, dadurch gekennzeichnet, daß man einen Tracer verwendet, der aus der Kupplung mit nativen, abgewandelten, synthetischen oder rekombinanten Formen des aFGF hervorgeht.

6. Verfahren nach einem der Ansprüche 1 bis 5, dadurch gekennzeichnet, daß man polyklonale und/oder monoklonale, spezifisch die aFGFs erkennende Antikörper verwendet.

7. Verfahren nach einem der Ansprüche 1 bis 6, dadurch gekennzeichnet, daß man die Bestimmung bei einer Ionenstärke von ungefähr 1 M NaCl durchführt.

8. Verfahren nach einem der Ansprüche 1 bis 7, dadurch gekennzeichnet, daß der Antikörper selbst gebunden ist und dann als Marker verwendet wird, wobei der aFGF selbst markiert ist oder nicht.

9. Verwendung des aFGF als biologischer, für pathologische Zustände charakteristischer Marker, besonders in den Bereichen der Kanzerologie und der entzündlichen und degenerativen Krankheiten.

10. Verfahren zur Erkennung pathologischer Zustände, insbesondere derjenigen, die auf wuchernde, degenerative oder entzündliche Krankheiten zurückzuführen sind, wobei man die Bestimmung von aFGF in einer biologischen Flüssigkeits- oder Gewebeprobe, die ihn enthalten kann, vornimmt, und wobei die so bestimmte Menge an aFGF einen repräsentativen Wert dieses pathologischen Zustands darstellt.

11. Kit mit Reagenzien zur Durchführung des Verfahrens nach einem der Ansprüche 1 bis 8 oder zur Anwendung nach Anspruch 9, wobei das Kit umfaßt:
- einen aus der Kopplung des aFGF mit einem Marker resultierenden Tracer,
- eine Bezugssubstanz, bestehend aus nativem, abgewandelten, synthetischem oder rekombinantem aFGF in Form einer Standardlösung von aFGF mit einer definierten Konzentration zur Erstellung einer Eichkurve,
- polyklonale und/oder monoklonale Antikörper, die spezifisch den aFGF erkennen.

12. Kit mit Reagenzien zur Durchführung des Verfahrens nach einem der Ansprüche 1 bis 8 oder zur Anwendung nach Anspruch 9, wobei das Kit umfaßt:
- einen Tracer, resultierend aus der Kopplung polyklonaler und/oder monoklonaler Antikörper, die spezifisch den aFGF erkennen, mit einem Marker,
- polyklonale und/oder monoklonale, nicht markierte Antikörper, die spezifisch den aFGF erkennen,
- eine Standardlösung des aFGF mit einer definierten Konzentration zur Erstellung einer Eichkurve.

13. Kit nach einem der Ansprüche 11 oder 12, dadurch gekennzeichnet, daß die Reagenzien bereitgestellt werden, um die Bestimmung in flüssiger oder fester Phase durchzuführen.

## Claims

1. Process for assaying the acidic from of FGF (aFGF, acidic fibroblast growth factor) and its immunoequivalents in biological fluids or tissues, wherein a tracer is used in conjugation with the said acidic form, which tracer can be recognized by antibodies specific to this same form, characterized in that assay is performed at a ionic strength at least equal to 0.7 M NaCl, which allows the dissociation of complexed aFGF forms present in the said biological fluids and tissues.

2. Process of claim 1, characterized by the use of an enzyme immunoassay technique.

3. Process of either claim 1 or 2, characterized in that the tracer results from the coupling of aFGF with an enzymatic marker such as acetylcholine esterase.

4. Process of claim 1, characterized by the use of a radioactive luminescent or magnetic marker.

5. Process of any one of claims 1 to 4, characterized by the use of a tracer resulting from the coupling with native, truncated, synthetic or recombinant forms of aFGF.

6. Process of any one of claims 1 to 5, characterized by the use of polyclonal and/or monoclonal antibodies specifically recognizing the aFGFs.

7. Process of any one of claims 1 to 6, characterized in that the assay is performed at a ionic strenght close to 1 M Nacl.

8. Process of any one of claims 1 to 7, characterized by the conjugation of the antibody itself which is then being used as a marker - aFGF being in such a case labeled or unlabeled.

9. Use of aFGF as a biological marker typical of pathological conditions, particularly in cancerology, degenerative and inflammatory disorders.

10. Method for recognizing pathological conditions especially due to proliferative, degenerative or inflammatory diseases, wherein an assay is performed on a sample of biological fluid or tissue, to measure its potential aFGF content, the resulting aFGF amount being a value representative of the said pathological condition.

11. Kit of reagents for carrying out the process of any one of claims 1 to 8, or for the above-mentioned application in accordance with claim 9, the said kit containing:
- a tracer resulting from the coupling of the aFGF with a marker,
- a competitor consisting in native, truncated, synthetic or recombinant aFGF under the form of a standard aFGF solution with a concentration determined for the elaboration of a standard curve,
- polyclonal and/or monoclonal antibodies, specifically recognizing the aFGF.

12. Kit of reagents for carrying out the process of any one of claims 1 to 8, or for the above-mentioned application in accordance with claim 9, the said kit containing:
- a tracer resulting from coupling with a marker of polyclonal and/or monoclonal antibodies, specifically recognizing the aFGF,
- polyclonal and/or monoclonal unlabeled antibodies, specifically recognizing the aFGF,
- a standard aFGF solution with a concentration determined for the elaboration of a standard curve.

13. Kit of either claim 11 or 12,characterized in that the reagents are so provided as to allow a solide-phase or a liquid-phase assay.
